# EUROPEAN PATENT APPLICATION

(11) **EP 4 520 358 A1**
(43) Date of publication of application: **12.03.2025**
(21) Application number: 23195883.6
(22) Date of filing: 07.09.2023
(51) Int. Cl.: A61L 29/04, A61L 29/14, A61L 29/08

(54) **HYDROPHILIC URINARY CATHETER**

(71) Applicant: Wellspect AB, 431 21 Mölndal (SE)
(72) Inventor: WESTLING, Åsa, 432 37 Lindome (SE)
(74) Representative: Taor, Simon Edward William

(57) **Abstract**

A urinary catheter comprises a catheter shaft and a discharge element, such as a flared funnel, and a lumen extending between drainage eyelets at a proximal insertion end of the catheter shaft and an opposite distal discharge end at the discharge element. The catheter is hydrophilic, and the catheter shaft comprises a hydrophilic coating provided on the substrate forming the shaft. The substrate is formed of a material comprising a blend of 70-99 wt% of a plastomer and 1-30 wt% of polyolefin, the polyolefin being at least one of polyethylene and polypropylene. The substrate material has been found to have good chemical and mechanical properties, and can at the same time be produced in an environmentally preferable, sustainable way, providing a low carbon footprint.

## Description

### TECHNICAL FIELD OF THE INVENTION

The present invention relates to a hydrophilic urinary catheter.

### BACKGROUND OF THE INVENTION

Many medical devices incorporate elongate shafts such as tubes which are intended for insertion into and through passageways of a living body such as those of the urethral tract and the cardiovascular system. The most common type of this general grouping of medical devices are known as catheters, such as urinary catheters.

Because of the intended use of urinary catheters certain parameters need to be satisfied by the material from which the elongate shaft is manufactured. The material must fulfill such requirements as softness, good kink resistance, good dimensional stability, processability, for example ease to form and/or glue, and the possibility to be sterilized by radiation, steam, ethylene oxide or other means. For hydrophilic urinary catheters, there is further the need for the material to accept and adhere to a hydrophilic coating which will impart desired hydrophilicity to the catheter. To this latter end, the chemistry of the substrate material is critical since this affects the possibility to coat the substrate.

In previous years, polyvinyl chloride (PVC) has been used to manufacture medical devices having elongate shafts for insertion into a body passageway such as catheters due to PVC fulfilling the requirements mentioned in the preceding paragraph. For instance, EP 0 093 093 by the same applicant makes known a process for manufacturing a PVC urinary catheter having a hydrophilic outer surface coating which exhibits a low coefficient of friction when wetted.

However, the suitability of PVC as a substrate for urinary catheters, and also many other previously proposed and used materials, is now being questioned on environmental grounds and further also because of the toxicity of certain constituents used in production, e.g. the plasticizers added to PVC.

Other substrate materials that have been previously proposed are for example to use a polyether block amide and a styrene block copolymer as substrate material for a hydrophilic catheter (WO 97/49437 by the same applicant). These materials have proven to be suitable for hydrophilic coating, and to have adequate mechanical and chemical properties. However, a problem with these materials is that they are relatively expensive to manufacture. Further, polyether block amide has relatively high resilience, which makes it unsuitable for certain applications. For example, catheters made of this material may be difficult to handle for disabled patients. When using styrene block copolymer, the adherence of surface coatings, such as hydrophilic coatings, is lower than when using e.g. polyether block amide.

US 2007/016169, also by the same applicant, proposed to use a blend of polyolefin together with a compound having active hydrogens as a material for a catheter substrate.

Further, WO 2019/113077 discloses a catheter substrate material having mechanical properties, such as stiffness and flexibility, which are stable over a large range of temperatures.

However, there is still a general problem for most previously known catheter substrates, that they are costly and/or harmful to the environment, and/or that there are problems related to the hydrophilic coating, such as too poor water retention properties, especially after leaching, too poor adherence to the substrate and too high friction of the hydrophilic surface when wetted. Further, alternatively, or additionally, the mechanical properties of the substrates may be inadequate, such as being too stiff or having too high resilience.

There is therefore a need for a new substrate material for a hydrophilic urinary catheter which is sustainable and even more environmentally acceptable and cost effective, to which the hydrophilic coating can be adequately adhered, and which has adequate mechanical and chemical properties.

### SUMMARY OF THE INVENTION

It is therefore an object of the present invention to address the above-discussed needs, and to overcome or alleviate all, or at least some, of the above-discussed problems. Specifically, it is an object of the present invention to provide a hydrophilic urinary catheter with a substrate material which is sustainable and environmentally preferable, and in particular having a low carbon footprint, which is cost-efficient, and which has adequate mechanical properties for use as a urinary catheter.

This object is obtained by a urinary catheter as defined in the appended claims.

According to an aspect of the present invention, there is provided a urinary catheter comprising a catheter shaft and a discharge element, a lumen extending between drainage eyelets at a proximal insertion end of the catheter shaft and an opposite distal discharge end at the discharge element, the catheter shaft comprising a substrate and a hydrophilic coating provided on the substrate, wherein the substrate is formed of a material comprising a blend of 70-99 wt% of a plastomer and 1-30 wt% of polyolefin, the polyolefin being at least one of polyethylene and polypropylene.

In the present application, the term "proximal" is used to indicate the end or portion of a catheter that is inserted into the body of the user, i.e. the end or portion of the catheter shaft that during use is closer in proximity to the user's body and/or initially enters the user's body upon insertion. The term "distal" is used to refer to an end or portion of the catheter that is opposite the proximal end or portion and is typically further away from the user's body. For the sake of consistency, when the terms "distal" and "proximal" are used in the context of other components, such as a package and the parts thereof, which are not intended for introduction into the user's body, "proximal" refers to the end or portion that is closer to the proximal end of the catheter within the assembly, while "distal" refers to an end or portion located opposite to such proximal end or portion.

"Plastomer" is, in the context of this application, to be understood in a broad sense, as any polyolefin plastomeric material which, alone or when mixed with another polymer, such as another polyolefin, combines qualities of elastomers and plastics, such as rubber-like properties with the processing ability of plastic. Plastomers include, but are not limited to, ethylene-alpha olefin copolymers and polybutylene (polybutene-1, poly(1-butene), PB-1). Suitable alfa olefins may be 1-butene, 1-pentene, 1-hexene, etc. The plastomers generally have a high compatibility with polyolefins, and when mixed with a more rigid polyolefin renders the mixture softer.

Plastomers, as well as polyethylene and polypropylene, are producible in a very environmentally preferable, sustainable, way, having a low carbon footprint, and are also relatively inexpensive. These materials are also available from renewable or recycled feedstock, making them available i.e. via a mass balance approach, thereby making it possible to reduce the carbon footprint even further. This type of material can further be easily recycled in conventional polyolefin recycling streams.

Thus, the carbon footprint is hereby lowered in comparison with carbon footprint of traditional elastomers, as the substrate now only comprises carbons and hydrogens and are polyolefins. Also, the costs for the material as well as manufacturing of catheters are lowered dramatically. Another advantage over the traditional elastomers is the chemical homogeneity of the new elastomers, as the components are based on olefine(s), whereas traditional elastomers comprise e.g. heteroatoms and/or cyclic moieties such as styrene moieties.

It has surprisingly been found that a blend of plastomer and at least one of polyethylene and polypropylene can be efficiently coated with a hydrophilic coating. The hydrophilic coating adheres well to the substrate surface. Further, the hydrophilic coating has excellent properties for use in urinary catheters, such as low friction when wetted, good water retention, good adherence to the substrate, etc.

Adherence of the hydrophilic coating may be further improved by use of one or more surface treatment processes prior to coating. In preferred embodiments the catheter substrate is treated with one or more of the following surface treatments prior to coating: plasma surface treatment, corona surface treatment, flame surface treatment and chemical surface treatment, such as by coating with a primer solution.

The hydrophilic coating is preferably arranged to provide a low-friction surface character of the urinary catheter when wetted by a wetting fluid, such as water or saline. The hydrophilic coating is preferably arranged to cover at least the entire insertable length of the catheter.

It has further been found that by suitably balancing the relative amount of plastomer(s) in the blend, the mechanical properties can be tailored to be suitable for use in substrates for urinary catheters. A urinary catheter needs to be rigid and stiff enough to enable insertion into the urethra by pushing it from the rear end. At the same time, the urinary catheter needs to be flexible enough to pass through the various curves and restricted areas of the urethra.

In particular, the hydrophilic urinary catheter may be a male urinary catheter, i.e. a catheter dimensioned and adapted for use by male users.

Catheterization, in particular for male users, can often be complicated. The male urethra is long, typically 15-29 cm, and comprises a number of curves and restricted areas through which it may be difficult to insert a catheter. On the one hand, it is generally a desire to make the catheter as stiff and rigid as possible since this makes it easier to push and control/manipulate the catheter from the rear end during insertion. A stiff catheter will also resist kinking, buckling and other deformations. However, a stiff catheter will have difficulty in finding its way and navigating around the narrow and restricted passages in the urethra, and may also cause pain and injury to the user. Thus, from this point of view, the catheter should instead be made as soft as possible. The stiffness and softness of the substrate consequently need to be finely balanced, to provide a urinary catheter which has stiffness and rigidity to obtain adequate maneuverability and at the same time a softness and flexibility to avoid pain, discomfort and injury on the user or patient.

The male urethra generally comprises, seen from the tip of the penis - the external urethral meatus - and in the direction towards the bladder, four sections. A first section may be referred to the penile part. This part is relatively straight and uncomplicated from a catheter insertion point of view. Next, there is the bulbous part, which is separated from the penile part by suspensory ligament. This transition may sometimes be problematic in view of catheterization. After the bulbous part follows the membranous part, in which the urethra performs a sharp bend, and where the external sphincter, the urogenital diaphragm, is located. This part, which is relatively short (1-2 cm) is often the most difficult to pass for a catheter. Following the membranous part, the prostatic part (sometimes subdivided into prostatic urethra and pre-prostatic urethra) leads past the prostate and ends with the internal sphincter at the bladder neck. This part may for some users also be difficult to pass for a catheter.

The words "soft"/"'softer" and "stiff "/"stiffer" here means relatively more flexible and relatively more rigid, respectively. Thus, soft/softer generally indicates a lower Shore A or micro Shore A value, a lower bending stiffness and/or a lower tensile modulus, whereas stiff/stiffer generally indicates a higher Shore A or micro Shore A value, a higher bending stiffness and/or a higher tensile modulus.

It has surprisingly been found that a urinary catheter with a substrate formed of a material comprising a blend of 70-99 wt% of a plastomer and 1-30 wt% of polyolefin, the polyolefin being at least one of polyethylene and polypropylene, provides an excellent balance between softness and stiffness, thereby providing excellent properties for the catheter. In particular, this substrate is much more flexible and softer than substrates made only by polyethylene and/or polypropylene.

In particular, it has been found that a substrate comprising a blend of about 80-85 wt% of plastomer and about 15-20 wt% of polyolefin has mechanical properties very similar to the well-known POBE material, a thermoplastic elastomer (TPE) of SEBS-type, used in the commercially available LoFric urinary catheters, produced and sold by Wellspect AB, Sweden. The LoFric POBE catheters have been in extensive use for many years and have proven excellent mechanical properties. This is e.g. shown in the articles "Evaluation of a new PVC-free catheter material for intermittent catheterization: a prospective, randomized, crossover study" by K. Johansson et al, Scand. J. Urol. Feb, 2013, 47(1):33-7, and "A multicenter, double-blind, randomized, parallel group study comparing polyvinyl chloride and polyvinyl chloride-free catheter materials" by J. A. Witjes et al, J. Urol. Dec. 2009, 182(6):2794-8.

Further, as already noted, it has been found that the substrate material has excellent chemical properties to be coated with a hydrophilic coating. The hydrophilic coating, when applied on the new substrate material, has a very high slipperiness which was also preserved after ageing. Both friction and water retention values were good and in level with the above-discussed LoFric POBE catheters, both before and after ageing.

In a preferred embodiment, the substrate is formed of a material comprising a blend of 75-85 wt% of plastomer and 15-25 wt% of polyethylene and/or polypropylene, such as about 80-85 wt% of plastomer and 15-20 wt% of polyethylene and/or polypropylene.

The substrate preferably has a hardness Shore A in the range 75 - 85, and preferably within the range 78-82.

The substrate may have a radiation resistance such that it can endure at least 50 kGy essentially without degradation.

The hydrophilic coating preferably comprises a hydrophilic polymer.

The hydrophilic coating may comprise at least one of: polyvinyl compounds, polylactames, in particular such as polyvinyl pyrrolidones, polysaccharides, in particular heparin, dextran, xanthan gum, derivatised polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride, polyactide, polyethylene glycol and copolymers thereof.

Preferably, the hydrophilic polymer is polyvinyl pyrrolidone (PVP).

The hydrophilic coating may form a polyurea network, and most preferably the polyurea network is arranged to form a covalent bond to active hydrogen groups in the substrate. Such a coating may be provided by applying sequentially to the surface of the substrate first a solution comprising between 0.05 to 40 percent (weight to volume) of an isocyanate compound and thereafter a solution containing between 0.5 and 50 percent (weight to volume) of polyvinylpyrrolidone and curing at an elevated temperature.

However, other hydrophilic coatings are also feasible, such as a coating comprising hydrophilic polymers cross-linked directly to the substrate. The cross-linking may be effected by means of irradiation, e.g. by electron beams or UV light. Such crosslinked coatings are e.g. known from US 8377498 by the same applicant, said document hereby being incorporated by reference in its entirety.

In a preferred embodiment, the hydrophilic polymer is cross-linked to the substrate, and most preferably by UV irradiation.

A high concentration of plastomer in the blend may result in a somewhat sticky surface. However, to the extent that this would occur, this problem may be alleviated, and the stickiness be reduced by addition of one or more slip agents to the blend. Slip agents reduce the coefficient of friction of the material to which they are added. Slip agents are per se known in the art. Traditional slip agents have a designed incompatibility with the bulk polymer, so that they are not bonded into the bulk but are free to migrate. For example, silica and/or fatty amides may be used as slip agents in this context.

Thus, in an embodiment the material of the substrate comprises a slip agent, and preferably in the range of 0.01-3 wt%, and more preferably 0.2-2 wt%.

The urinary catheter is preferably a urinary catheter for intermittent catheterization, i.e. for short time use, such as catheterization of a few minutes' duration, being repeated a number of times each day. The term "short term use" indicates a use that is limited in time, and in particular limited to a time period of less than 15 minutes, and preferably less than 10 minutes, and most preferably less than 5 minutes. The catheter of the present invention is excellently suited for self-catheterization and can safely and easily be used also by inexperienced users, and/or users suffering from poor dexterity. The urinary catheter can be used both for female and male catheters. However, due to its advantageous properties the urinary catheter is particularly suited for male catheters, preferably having a total length within the range of 35-40 cm, and having an insertable length within the range of 20-35 cm.

The discharge element may be flared and formed as a funnel. The discharge element could be made of the same material as the substrate, and may e.g. be monolithically integrated with the substrate. However, alternatively, the discharge element may be formed by a different material, and may in such embodiments e.g. be formed as a separate piece, connected with the catheter shaft e.g. by adhesion.

The tip may be straight, extending in the same direction as the tubular shaft and forming a rounded forward end, thereby forming a Nelaton type catheter. However, the tip may also be curved, forming a Tiemann or Coude type catheter. Tiemann and Coude catheters have a tip which is angled upward, to assist in negotiating the male prostatic curve. Thus, this tip form facilitates passage through the bladder neck in the presence of obstruction e.g. from a slightly enlarged prostate gland (e.g. in benign prostatic hyperplasia), and can be helpful for such and other difficult insertions.

The tip may be formed directly in the forward end of the tubular shaft, e.g. by melting. However, it is also possible to provide a separately manufactured tip, of the same or a different material, and subsequently attaching this tip to the forward end of the catheter shaft. Such a separately manufactured tip may be manufactured by injection molding, but may also be manufactured in other ways. A separately produced tip may be connected to the tubular shaft by at least one of welding, adhesion, and injection molding.

The catheter shaft is preferably formed by extrusion.

The plastomer in the substrate material may comprise an ethylene-alpha olefin copolymer. For example, the alpha olefin may comprise at least one of 1-butene, 1-hexene and 1-octene. In a preferred embodiment, the plastomer is an ethylene-based octene-1 copolymer. In another embodiment, the plastomer is polybutene-1 (PB-1).

In an embodiment, the substrate is formed of a material comprising a blend of 70-90 wt% of polybutene-1 and 10-30 wt% of polypropylene, and preferably 75-85 wt% of polybutene-1 and 15-25 wt% of polypropylene.

In another embodiment, the substrate is formed of a material comprising a blend of 70-90 wt% of an ethylene-alpha olefin copolymer, such as an ethylene-based octene-1 copolymer, and 10-30 wt% of polyethylene, and preferably 75-85 wt% of an ethylene-alpha olefin copolymer and 15-25 wt% of polyethylene.

In an embodiment, the plastomer comprises only an ethylene-alpha olefin copolymer. In another embodiment, the plastomer comprises only polybutene-1. In another embodiment, the plastomer comprises both an ethylene-alpha olefin copolymer and polybutene-1.

In an embodiment, the polyolefin comprises only polyethylene. In another embodiment, the polyolefin comprises only polypropylene. In another embodiment, the polyolefin comprises both polyethylene and polypropylene.

The urinary catheter may further be included in a urinary catheter assembly, which may further comprise a package, arranged to accommodate at least the insertable part of the catheter. In embodiments, the package may enclose and accommodate the entire catheter.

According to an aspect, there is provided a urinary catheter assembly comprising the urinary catheter as discussed in the foregoing and a package accommodating the urinary catheter, wherein the package is made of a material comprising a polyolefin, and preferably the same type of polyolefin as in the urinary catheter or materials that are compatible in the recycling stream. In particular, the polyolefin may be at least one of polyethylene and polypropylene. Hereby, the package could be recycled in the same recycling path as the catheter. Preferably, the entire urinary catheter assembly could be recycled in the same recycling path. This facilitates handling of the catheter assembly for the user, and also facilitates recycling for the producer, and makes the recycling more efficient.

The package material may comprise at least 50 wt% of polyolefin, and in particular polyethylene and/or polypropylene. In an embodiment, the package material comprises essentially only polyethylene and/or polypropylene. In other embodiments, the package material comprises a blend of a plastomer and at least one of polyethylene and polypropylene. The weight proportions of the constituents in the blend may in an embodiment be essentially the same as in the catheter substrate material.

The package may also comprise a supply of wetting fluid, for wetting of the hydrophilic coating of the catheter. In an embodiment, the wetting fluid may be arranged in direct contact with the catheter, thereby maintaining the catheter in an activated, ready to use, state. Alternatively, the wetting fluid may be arranged in a separate compartment of the package, such as in a separate sachet arranged within the package. Hereby, the wetting fluid may be released at a suitable time prior to use, such as immediately before the intended use. Opening of the wetting fluid compartment for release of the wetting fluid can be made by e.g. applying a pressure on the wetting fluid compartment, such as by squeezing or pinching the sachet. However, other ways of opening the compartment are also feasible, as is per se well known in the art, such as by twisting, pulling, pushing, etc. According to yet another embodiment, the wetting fluid may be arranged in a separate compartment arranged in such a way that the catheter shaft can be moved through the compartment to be wetted. Such a compartment may e.g. be arranged so that the catheter shaft is pulled through the compartment upon extraction from a package.

The wetting fluid is preferably in the form of a wetting liquid, and can e.g. be sterile water or saline. However, other fluids, such as vapor, may also be used.

The wetting fluid compartment may be provided as a separate part from the package, and either be loosely arranged in the package, or be connected to the package. Alternatively, the wetting fluid compartment may form an integrated part of the package.

The package may be made of a foil, which is welded along the sides. For this purpose, materials like poly-ethylene-terephthalate (PETP), low-density poly-ethylene (LDPE), high-density poly-ethylene (HDPE), poly-propylene (PP), poly-amide (PA), amorphous polyester (PET) and surface-treated paper may be used. However, other materials may also be used, as is per se well known in the art.

The urinary catheter is preferably maintained sterile when arranged in the package. Hereby, the catheter is completely sterile when it exits the package, thereby reducing the risk of getting infected when used. To this end, the catheter assembly may when once assembled, or previously in the assembling process, be irradiated by electron beam radiation, and thus sterilized.

According to another aspect of the invention, there is provided a urinary catheter comprising a catheter shaft and a discharge element, a lumen extending between drainage eyelets at a proximal insertion end of the catheter shaft and an opposite distal discharge end at the discharge element, the catheter shaft comprising a substrate and a hydrophilic coating provided on the substrate, wherein the substrate is formed of a material comprising at least 80 wt% of plastomer.

The urinary catheter of such embodiments provides similar advantages and properties as in the above-discussed embodiments.

In accordance with this aspect, the substrate may be formed of a material comprising at least 85 wt% of plastomer, and preferably at least 90 wt% of plastomer, or even more preferably at least 95 wt% of plastomer, and most preferably at least 99 wt% of plastomer. The material may be a blend of plastomer and a polyolefin, such as polyethylene and/or polypropylene. However, the material may also be essentially pure plastomer. In an embodiment, the substrate may be formed of a material comprising essentially 100 wt% of plastomer. The plastomer may be a single plastomer, or a blend of two or more different plastomers.

### BRIEF DESCRIPTION OF THE DRAWINGS

The present invention will be described in more detail with reference to the appended drawings, showing currently preferred embodiments of the invention.
Fig. 1 is a sideview of an intermittent urinary catheter in accordance with an embodiment.
Fig. 2 is a sideview of an intermittent urinary catheter assembly in accordance with an embodiment, comprising the urinary catheter of Fig. 1 arranged in a package.

### DETAILED DESCRIPTION OF CURRENTLY PREFERRED EMBODIMENTS

In the following detailed description preferred embodiments of the invention will be described. However, it is to be understood that features of the different embodiments are exchangeable between the embodiments and may be combined in different ways, unless anything else is specifically indicated. It may also be noted that, for the sake of clarity, the dimensions of certain components illustrated in the drawings may differ from the corresponding dimensions in real-life implementations. Even though in the following description, numerous specific details are set forth to provide a more thorough understanding of the present invention, it will be apparent to one skilled in the art that the present invention may be practiced without these specific details. In other instances, well known constructions or functions are not described in detail, so as not to obscure the present invention.

The following discussion is in particular concerned with male hydrophilic urinary catheters for intermittent use. However, the invention can also be used in relation to other types of urinary catheters, such as female urinary catheters and/or indwelling urinary catheters.

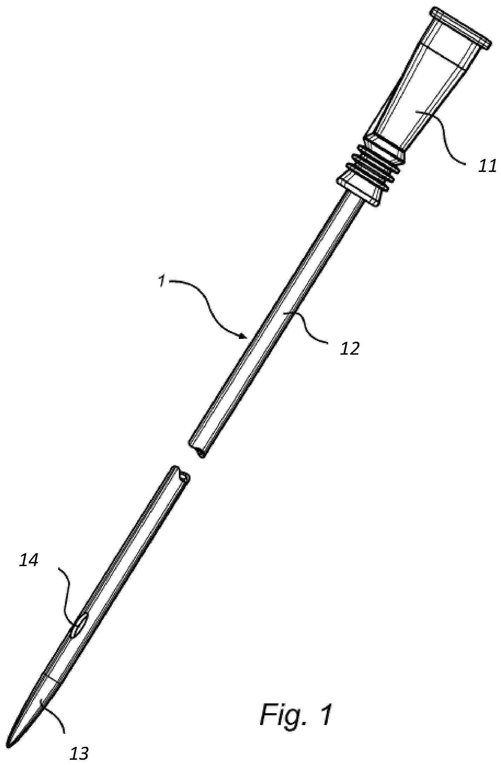

A catheter 1 as illustrated in Fig. 1, comprises a flared rearward portion 11, shaped as a funnel, and an elongate shaft or tube 12 projecting forwardly from the rearward portion 11. An open-ended internal lumen extends from the rear end of the rearward portion 11 to one or more drainage apertures 14 in a forward end of the catheter. At the forward end of the elongate shaft 12, a tapering tip 13 is arranged, having a rounded tip end. The rearward portion 11 may function as a connector of the catheter 1, being connectable to other devices, such as a urine collection bag, a drainage tube or the like.

At least a part of the elongate tube 12 forms an insertable length to be inserted through a body opening of the user, such as the urethra in case of a urinary catheter. By insertable length is normally meant that length of the elongate tube 12 which is insertable into the urethra of the patient during ordinary use. A hydrophilic coating is provided on the catheter, and preferably at least the insertable length is coated with a hydrophilic polymer, such as PVP. Typically, the insertable length is 60-130 mm for a female patient and 200-350 mm for a male patient.

The tip 13 may be straight, extending in the same direction as the tubular shaft and forming a rounded forward end, thereby forming a Nelaton type catheter. Such an embodiment is illustrated in Fig. 1. The tip is preferably arranged conically tapering in the forward direction, to end in a rounded tip. However, alternative configurations are also feasible. For example, the tip may have parts being slightly enlarged, such as a ball shaped forward end, or the like. For example, the end may be provided with a slightly enlarged rounded or ball-shaped head portion.

The tip may also be curved, forming a Tiemann or Coude type catheter.

The tips may be designed and connected to the tubular shaft in various ways, or be formed as an integrated, and preferably monolithically integrated, part of the catheter shaft.

The connector, i.e. the flared rearward portion 11 is optional, and catheters without any flared rearward portion may also be used. In case a flared connector is used, this may be formed integrally and monolithically at the rearward end of the tubular shaft. However, it may also be formed as a separate component, being connected to the tubular shaft by means of welding, adhesion or the like. It may also be injection molded directly in place. In this case, injection molding of the tip at the forward end of the tubular shaft and the connector at the rearward end of the tubular shaft can be performed simultaneously.

The catheter is coated with a hydrophilic coating, as has been discussed in the foregoing. In particular, it is preferred to have the hydrophilic coating arranged on the outer surface of the tip and the catheter shaft, and preferably at least over the insertable part. Many different types of well-known hydrophilic surfaces can be used.

The urinary catheter may further be included in a urinary catheter assembly, which may further comprise a package, arranged to accommodate at least the insertable part of the catheter. In embodiments, the package may enclose and accommodate the entire catheter.

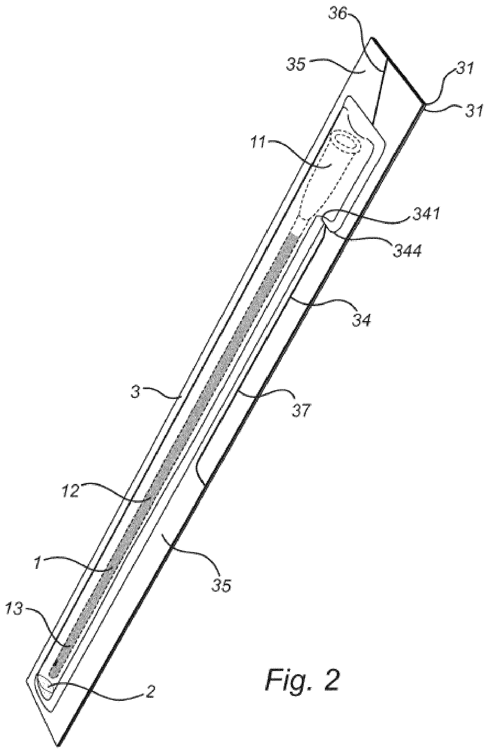

The package may be realized in many ways, as is per se known in the art. An example of a specific package will be discussed in the following, with reference to Fig. 2. This package is generally of the type disclosed in US 10 569 046 and US 10 905 849, both by the same applicant, said documents hereby being incorporated in their entirety by reference. However, even though this type of package is highly advantageous, many other types of packages may be used instead, having e.g. a tubular compartment, having peel opening(s), etc. As one example, a package as disclosed in US 6 848 574, also by the same applicant, may be used.

The package disclosed in relation to the illustrative example has a rectangular, elongate shape. However, the package may also have other shapes, such as being of a square shape. In an embodiment, the package may also function as a urine collection bag during catheterization or contain a separate urine collection bag.

The catheter assembly as illustrated in Fig. 2 comprises a catheter 1 as discussed in the foregoing and a package 3 accommodating the catheter, and optionally a wetting fluid 2 arranged in the package. The wetting fluid may be arranged directly in contact with the catheter, or arranged separately in a separate compartment. The wetting fluid is preferably a wetting liquid, and preferably an aqueous liquid, such as water or saline. However, the wetting liquid may in addition comprise one or more additives.

The package may be made of sheet material but may also be made by injection molding. In the shown embodiment, the package is made of two sheet materials 31, connected around the edges to form an inner cavity housing the catheter and the optional wetting fluid. The first and second sheet materials are preferably connected around the edges by means of welding, forming welded edge joints. However, alternatively, a folded sheet material may also be used for forming the package, whereby one or several sides of the package may be closed by the fold instead of, or in addition to, the weld. The sheet material may also be provided in the form of a tube, requiring even less welded joints to close the package. However, depending on which sides that are closed, additional welds along the closed sides may still be required to form the tear lines, protrusions, etc, as discussed in the following.

The sheet material is preferably a tearable and/or peelable material, and preferably comprises a laminated sheet material, and preferably having a weldable inner layer and a protective outer layer.

Further, the package is preferably made of a material having low gas permeability, and preferably being entirely gas-impermeable.

The sheet materials are preferably of a flexible plastics material. The material may be transparent, but opaque or semi-opaque materials may also be used. For example, the packaging material can be made of polymer materials such as polyethylene (PE), polypropylene (PP), polyamide (PA), polyethylene terephthalate) (PET), oriented polypropylene (OPP), oriented polyamide (OPA), etc. Also, the receptacle can be made of one or several materials functioning as barrier material or having low water vapor transmission. The barrier material may e.g. be provided as a metallization layer on a substrate sheet. The material of the sheet material, or the material of one of the layers in case of a laminate or with a deposited barrier layer, may to this end comprise or consist of one or several of aluminum, aluminum oxide, silicone oxide, metallocene polyvinylidene chloride (PVdC) and poly(ethylene-vinylalcohol) (EVOH). For example, the flexible material can be made as coextruded polyolefines with polyamides, poly(ethylene terephthalate) (PET), including barrier resins such as polyvinylidene chloride (PVdC) or poly(ethylene-vinylalcohol) (EVOH). However, other materials exhibiting similar properties are also feasible.

The package is preferably made of a recyclable packaging material. In an embodiment the packaging material comprises a polyolefin, and particularly at least one of polyethylene and polypropylene. The packaging material is preferably a mono-material, comprising essentially only one type of material, such as only polyethylene or polypropylene. However, the packaging material may also be a combination of compatible materials for recycling, i.e. materials that are compatible in a recycling stream. The polyolefin material of the package is preferably the same polyolefin material as also included in the urinary catheter. Alternatively, the package may comprise a blend of a plastomer and a polyolefin, and preferably essentially the same blend as used in the catheter substrate material.

In the exemplary embodiment illustrated in the drawings, the package 3 is formed as an elongate package, comprising two longitudinal sides extending in a length direction of the package, and two short sides extending in a transversal direction of the package. All sides are closed, thereby forming a closed compartment accommodating the medical device in a sterile way. Preferably, the package is arranged to maintain sterility during a prolonged storage. The assembly preferably has a shelf life of at least 2 years, and most preferably at least 3 years, and even more preferably at least 5 years. In the exemplary embodiment, the package is provided with tear openings for enabling opening of the package and extraction of the catheter. However, many other ways of opening the package are also feasible, such as peel openings, openable caps or lids, etc. It is also possible to combine various opening mechanisms in one package.

The longitudinal sides and short sides may be closed in various ways, such as by welded joints, by folding, etc. In the illustrative example, a first weld 34 is shown, arranged along one longitudinal side, and a second weld 35 is arranged along one short side.

In the illustrative example, two different tear openings are provided: A first tear line 36 and a second tear line 37. When opening the package with the first tear line 36, the user grips the areas of the package arranged on both sides of the tear line 36, and pulls the gripping areas apart. Opening of the package may additionally, or alternatively, occur through the second tear line 37.

The elongate shaft/tube of the catheter is made of a substrate material. This substrate is made of a polymer blend of 70-90 wt% of a plastomer and 10-30 wt% of polyethylene and/or polypropylene. In a preferred embodiment, the substrate is formed of a material comprising a blend of 75-85 wt% of plastomer and 15-25 wt% of polyethylene and/or polypropylene, such as about 80 wt% of plastomer and 20 wt% of polyethylene and/or polypropylene.

The plastomer in the substrate material may comprise an ethylene-alpha olefin copolymer. For example, the alpha olefin may comprise at least one of 1-butene, 1-hexene and 1-octene. In an embodiment, the plastomer comprises an ethylene-based octene-1 copolymer, such as the plastomer Bormed PL8830-PH produced and sold by Borealis, or Exact 5061 produced and sold by ExxonMobile. In another embodiment, the plastomer comprises polybutene-1 Purell KT MR07 (PB-1) sold by LyondellBasell.

The polymer blend may further comprise a slip agent. Thus, in an embodiment, the material of the substrate comprises a slip agent, such as silica and/or a fatty amide, and preferably in the range of 0.01-3 wt%, and more preferably 0.2-2 wt%. It is of particular interest to include a slip agent in the polymer blend when the blend comprises 80 wt% or more of plastomer and/or when the plastomer comprises polybutene-1.

The polymer blend may also comprise other additives, such as an antibacterial or antimicrobial agent. Preferably, the total amount of additives, apart from plastomer, polyethylene and polypropylene, is less than 5 wt%, and preferably less than 3 wt%, and more preferably less than 2 wt%, and most preferably less than 1 wt%.

The blend may be obtained by physical blending, compounding or any other suitable blending method. Such blends may then be extruded or molded by any suitable methods to form a catheter tube.

In order to be suitable for use as a urinary catheter, and in particular for a male urinary catheter, it has been found that the substrate material should fulfill at least some of the following requirements, and preferably essentially all of them:
- The material should have a suitable Shore A hardness, preferably in the range 75 - 85, and most preferably within the range 78-82.
- The substrate should preferably be sterilizable by known sterilization methods. In particular it is preferred that the substrate has a radiation resistance such that it can endure at least 50 kGy essentially without significant degradation, in order to enable radiation sterilization of the medical device.
- The material should exhibit low resilience.
- The material should have good kinking properties.
- The polymer should preferably be extrudable, or usable for molding, and in particular useable for injection molding.
- The substrate material should preferably be biocompatible, and preferably available in medical grade quality.
- The material should preferably have good dimension stability. In particular, it is preferred that the longitudinal shrinkage of the catheters as a result of the coating process is less than 5 percent, and preferably less than 1 percent, of the original length.
- To summarize, the new substrate material should preferably have mechanical properties and a capability of being coated similar to the POBE material of the above-discussed commercially available LoFric POBE catheter. It has been found and experimentally verified, as will be discussed in the following, that the new material blend fulfills all these requirements.

The hydrophilic coating is preferably arranged to provide a low-friction surface character of the urinary catheter when wetted by a wetting fluid, such as water or saline. The hydrophilic coating is preferably arranged to cover at least the entire insertable length of the catheter.

The hydrophilic coating preferably comprises a hydrophilic polymer, and preferably polyvinyl pyrrolidone (PVP). In an embodiment, the PVP is PVP K90.

However, other hydrophilic polymers are also feasible, in addition, or as an alternative, to PVP. Thus, in embodiments, the hydrophilic coating may comprise at least one of: polyvinyl compounds, polylactames, in particular such as polyvinyl pyrrolidones, polysaccharides, in particular heparin, dextran, xanthan gum, derivatized polysaccharides, hydroxy propyl cellulose, methyl cellulose, polyurethanes, polyacrylates, polyhydroxyacrylates, polymethacrylates, polyacrylamides, polyalkylene oxides, in particular polyethylene oxides, polyvinyl alcohols, polyamides, polyacrylic acid, copolymers of the previously mentioned polymers, copolymers of vinyl compounds and acrylates or anhydrides, copolymers of vinylpyrrolidone and hydroxy ethylmethyl acrylate, cationic copolymers of polyvinyl pyrrolidone and copolymer of polymethylvinyl ether and maleinic acid anyhydride, polyactide, polyethylene glycol and copolymers thereof.

The hydrophilic coating may form a polyurea network. Such a PVP coating may be formed by a solvent based cross-linking system containing isocyanate and methylene chloride, generally of the type disclosed in EP 0093093, and also discussed in EP 2 177 238.

However, other hydrophilic coatings are also feasible, such as a coating comprising hydrophilic polymers cross-linked directly to the substrate. The cross-linking may be effected by means of irradiation, e.g. by electron beams or UV light. Such crosslinked coatings are e.g. known from US 8 377 498 by the same applicant, said document hereby being incorporated by reference in its entirety. A PVP coating crosslinked by UV radiation could be made in accordance with the method described in Example 1 in WO 2004/075944, with the exception of excluding NMP due to environmental and safety reasons. A PVP coating crosslinked by E-beam radiation could be made in accordance with the disclosure of EP 2 198 897.

In a preferred embodiment, the hydrophilic polymer is cross-linked to the substrate, and most preferably by UV irradiation.

### Experimental results

Catheters were prepared using different relative proportions of polyethylene ("PE", LDPE LE6600-PH from Borealis), polypropylene ("PP", Purell RP 270G from LyondellBasell), polybutene-1 ("PB", Purell KT MR07 from LyondellBasell) and ethylene-based octane-1 copolymer ("EO1", Bormed PL8830-PH from Borealis).

The catheter tubes were produced by extrusion.

The catheter shafts were coated with different types of hydrophilic coatings. One type of hydrophilic coating comprised a polyurea network, formed by an isocyanate compound, as discussed in the foregoing, and essentially corresponding to the coating discussed in EP 0093093 and EP 2 177 238. Another type of hydrophilic coating comprised a hydrophilic polymer cross-linked to the substrate by UV irradiation, as discussed in the foregoing, and essentially corresponding to the coating discussed in Example 1 in WO 2004/075944. A third type of hydrophilic coating comprised a hydrophilic polymer cross-linked to the substrate by electron beam irradiation, as also discussed in the foregoing, and essentially corresponding to the coating discussed in EP 2 198 897. All the hydrophilic coatings comprised PVP as the hydrophilic polymer.

The catheters were packed and sterilized by e-beam irradiation at 46 kGy.

Tests were made on the thus prepared catheters to evaluate both mechanical properties of the catheters shaft and properties of the hydrophilic coatings.

As comparative examples, catheters having the same dimensions and provided with the same coatings were prepared using the previously discussed POBE material, identical to the material used in the commercially available LoFric POBE catheters.

In addition, some properties of commercially available urinary catheters from other producers were also tested. These urinary catheters were Actreen Hi-Lite Cath from B. Braun Medical, Infyna Chic from Hollister Inc. and SpeediCath Standard from Coloplast.

In a first line of tests, the bending stiffness of the catheters were evaluated. The bending stiffness was measured in a three-point bending set-up where the two ends of the tube were placed horizontally, and the force applied to press a middle point of the tube downwards was measured using a tensile tester. Fmax is the maximum force obtained.

The results of these measurements are provided in the following table.

| Reference | Material | Bending stiffness Fmax (N) |
|---|---|---|
| Ex. 1 | EO1 (85 wt%) + PE (15 wt%) | 2.6 |
| Ex. 2 | EO1 (70 wt%) + PE (30 wt%) | 3.6 |
| Ex. 3 | EO1 (60 wt%) + PE (40 wt%) | 4.0 |
| Ex. 4 | EO1 (40 wt%) + PE (60 wt%) | 7.0 |
| Ex. 5 | PB (80 wt%) + PP (20 wt%) | 2.4 |
| Ex. 6 | PB (70 wt%) + PP (30 wt%) | 3.5 |
| Ex. 7 | PB (60 wt%) + PP (40 wt%) | 4.3 |
| Comp. 1 | LoFric POBE from Wellspect Healthcare | 2.2 |
| Comp. 2 | Actreen Hi-Lite Cath from B. Braun Medical | 3.1 |
| Comp. 3 | Infyna Chic from Hollister Inc. | 2.9 |
| Comp. 4 | SpeediCath Standard from Coloplast | 2.6 |

The LoFric POBE catheter (Comp. 1) can be seen as a benchmark, having documented good mechanical properties for use as a urinary catheter. It can be seen that the other tested commercially available catheters (Comp. 2-4) have similar bending stiffnesses, even though being stiffer than the LoFric POBE catheter (Comp. 1).

It can further be seen that for catheters having substrates made of a blend of EO1 and PE, Ex. 1 had a bending stiffness similar to Comp. 1, and the same or lower than for Comp. 2-4. Ex. 2 has a higher, but yet acceptable, bending stiffness, being slightly higher than e.g. Comp. 2. However, the bending stiffness of Ex. 3 and Ex. 4 is unacceptable, and these catheters are not suitable for use as urinary catheters, at least not for male urinary catheters.

Similarly, for catheters having substrates made of a blend of PB and PP, Ex. 5 had a bending stiffness almost as good as Comp. 1, and lower than for Comp. 2-4. Ex. 6 has a higher, but yet acceptable, bending stiffness, being slightly higher than e.g. Comp. 2. However, the bending stiffness of Ex. 7 is unacceptable, and this catheter is not suitable for use as a urinary catheter at least not for male urinary catheters.

From this it may be concluded that the plastomer content, such as EO1 or PB, in the substrate material blend need to be at least 70 wt% to be suitable for use as a catheter shaft, and in particular for use as a catheter shaft for male urinary catheters.

This was also confirmed in tests made on an artificial urethra made of silicon, arranged to simulate the pathway of a male urethra. Experienced users inserted the various catheter samples into the urethra. It was found that the samples Ex. 1, Ex. 2, Ex. 5 and Ex. 6 were as easy to insert as the LoFric POBE reference, i.e. Comp. 1, whereas Ex. 3, Ex. 4 and Ex. 7 required much more force and effort to insert, which, had it been a real urethra, would most likely have resulted in pain, and possibly also injury, for the user.

The resistance to kinking of the tubular catheter shafts was also tested and evaluated.

A measure on the kink resistance is the distance between the ends of the catheter tube when kinking occurs, in the following referred to as the kink distance, and the maximum force applied immediately prior to the kinking, in the following referred to as Fmax. The measurement data is provided in the following table.

| Reference | Kink distance (mm) | Fmax (N) |
|---|---|---|
| Ex. 1 | 18.16 | 1.10 |
| Ex. 2 | 17.96 | 1.31 |
| Ex. 3 | 16.37 | 1.61 |
| Ex. 4 | 16.22 | 2.85 |
| Ex. 5 | 18.57 | 1.18 |
| Ex. 6 | 18.28 | 1.19 |
| Ex. 7 | N/A | 1.71 |
| Comp. 1 | 17.49 | 0.99 |

Thus, it was found that all the preferred examples Ex. 1, Ex. 2, Ex. 5 and Ex. 6 had a kink resistance similar to the LoFric POBE reference, Comp. 1. The kink resistance of Ex. 3, Ex. 4 and Ex. 7 is much higher, which is again an indication that these catheter samples are too stiff to be useable as urinary catheters.

The coating properties were tested in a number of ways. The coatings were evaluated manually by a number of experienced test persons, to evaluate slipperiness and smoothness of the coated surfaces, both in wetted and dry states. It was found that the slipperiness and smoothness of the coated surfaces for all the exemplary embodiments Ex. 1-7, and for all the different hydrophilic coatings, were as good as the LoFric POBE reference (Comp. 1).

Adherence of the coatings was also tested with an abrasion test, wherein the coatings were subject to forceful rubbing. Also here, the coated surfaces for all the exemplary embodiments Ex. 1-7, and for all the different hydrophilic coatings, were found to be as good as the LoFric POBE reference (Comp. 1).

Further, the catheters of Ex. 1-7 with the different hydrophilic coatings were tested for water retention in ambient air. To this end, the catheters were wetted during 30 sec., and the water content (mg/cm2) in the hydrophilic coating was determined after 1 and 6 minutes, respectively. The water content was determined by weighing the catheters before wetting, to obtain a reference weight, wetting the catheters in water, thereafter measuring the catheters weight a certain time after wetting, and subtracting the reference weight from this subsequently measured weight. The obtained weight difference is a measure of the water content being held by the hydrophilic coating at the time of measurement.

It was found that the water retention for all the tested samples were similar to the LoFric POBE (Comp. 1) reference. The measured water retention after 1 minute were, for all the samples, in the range of 12-14 mg/cm², which is very similar to the water retention of 12.3 mg/cm² after 1 minute for LoFric POBE. The measured water retention after 6 minutes were, for all the samples, in the range of 7-9 mg/cm², which is very similar to the water retention of 7.5 mg/cm² after 6 minutes for LoFric POBE.

The friction of the coated surfaces was determined by a friction testing apparatus, a Harland friction tester, with a clamp force of 100 g and saddle-shaped clamps. The measured coefficient of friction was, for all the samples, in the range of 0.005-0.015, which is less than the coefficient of friction for LoFric POBE of about 0.020 using this set-up.

Based on these measurements and evaluations it can be concluded that the plastomer based substrates can be coated with a hydrophilic coating suitable for use in urinary catheters, and that these hydrophilic coatings have the same, or even better, properties and qualities than presently sold commercial hydrophilic urinary catheters.

### Concluding remarks

The present invention has now been disclosed with reference to specific exemplary embodiments. However, it will be acknowledged by the skilled addressee that several modifications are possible. For example, the urinary catheter may be arranged in other types of packages, different plastomers may be used in the substrate, different hydrophilic polymers may be used in the hydrophilic coating, etc.

The above-discussed and other obvious modifications must be considered to be within the scope of the present invention, as it is defined by the appended claims. It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting to the claim. The word "comprising" does not exclude the presence of other elements or steps than those listed in the claim. The word "a" or "an" preceding an element does not exclude the presence of a plurality of such elements.

## Claims

1. A urinary catheter comprising a catheter shaft and a discharge element, a lumen extending between drainage eyelets at a proximal insertion end of the catheter shaft and an opposite distal discharge end at the discharge element, the catheter shaft comprising a substrate and a hydrophilic coating provided on the substrate, wherein the substrate is formed of a material comprising a blend of 70-99 wt% of a plastomer and 1-30 wt% of polyolefin, the polyolefin being at least one of polyethylene and polypropylene.

2. The urinary catheter of claim 1, wherein the substrate is formed of a material comprising a blend of 70-90 wt% of plastomer, and preferably 75-85 wt%, and 10-30 wt% of polyethylene and/or polypropylene, and preferably 15-25 wt%.

3. The urinary catheter of claim 1 or 2, wherein the plastomer comprises an ethylene-alpha olefin copolymer.

4. The urinary catheter of claim 3, wherein the alpha olefin comprises at least one of 1-butene, 1-hexene and 1-octene, and preferably 1-butene.

5. The urinary catheter of any one of the preceding claims, wherein the plastomer comprises polybutene-1.

6. The urinary catheter of any one of the preceding claims, wherein the discharge element, preferably in the form of a flared funnel, is formed as an integrated, monolithic unit with the catheter shaft, and formed of the same material as the substrate.

7. The urinary catheter of any one of the preceding claims, wherein the hydrophilic coating comprises a hydrophilic polymer, and preferably polyvinylpyrrolidone.

8. The urinary catheter of any one of the preceding claims, wherein the material of the substrate further comprises a slip agent, and preferably in the range of 0.01-3 wt%, and more preferably 0.2-2 wt%.

9. The urinary catheter of claim 8, wherein the material of the substrate comprises 0.01-0.5 wt% of slip agent, and preferably in the range of 0. 1-0.3 wt%.

10. The urinary catheter of any one of the preceding claims, wherein the substrate is formed of a material comprising a blend of 70-90 wt% of polybutene-1 and 10-30 wt% of polypropylene, and preferably 75-85 wt% of polybutene-1 and 15-25 wt% of polypropylene.

11. The urinary catheter of any one of the claims 1-10, wherein the substrate is formed of a material comprising a blend of 70-90 wt% of ethylene-alpha olefin copolymer and 10-30 wt% of polyethylene, and preferably 75-85 wt% of an ethylene-alpha olefin copolymer and 15-25 wt% of polyethylene.

12. A urinary catheter assembly comprising the urinary catheter of any one of the claims 1-11 and a package accommodating the urinary catheter, wherein the package is made of a material comprising a polyolefin, and preferably the same polyolefin type as in the urinary catheter.

13. The urinary catheter assembly of claim 16, wherein the package is made of a material comprising at least one of polyethylene and polypropylene.

14. A urinary catheter comprising a catheter shaft and a discharge element, a lumen extending between drainage eyelets at a proximal insertion end of the catheter shaft and an opposite distal discharge end at the discharge element, the catheter shaft comprising a substrate and a hydrophilic coating provided on the substrate, wherein the substrate is formed of a material comprising at least 80 wt% of plastomer.

15. The urinary catheter of claim 14, wherein the substrate is formed of a material comprising at least 90 wt% of plastomer, and most preferably essentially 100 wt% of plastomer.
